# DEMANDE DE BREVET EUROPEEN

(11) **EP 1 153 625 A1**
(43) Date de publication de la demande: **14.11.2001**
(21) Numéro de dépôt: 00810396.2
(22) Date de dépôt: 09.05.2000
(51) Int. Cl.: A61M 5/32

(54) **Dispositif de sécurité pour la pointe d'une aiguille médicale**

(71) Demandeur: Pegaitaz, Raphäel, 1205 Genève (CH); Myers, Andrew, 1204 Geneve (CH)
(72) Inventeur: Pegaitaz, Raphäel, 1205 Geneve (CH)
(74) Mandataire: Gresset, Jean

(57) **Abrégé**

L'invention concerne un dispositif de sécurité (16) pour une aiguille médicale (10) dotée d'une gorge (18) ménagée à proximité de sa pointe (12). Il comporte:
- un organe tubulaire interne (20) déplaçable le long de l'aiguille jusqu'à sa pointe de manière à l'emprisonner et élastiquement déformable entre:
   . un état ouvert qu'il garde tant qu'il n'est pas au niveau de la pointe et pour lequel sa partie proximale (26), de diamètre interne réduit, enserre l'aiguille, et
   . un état fermé qu'il prend lorsqu'il arrive au niveau de la pointe et pour lequel sa partie proximale (26) se trouve dans la gorge (18), et
- un organe tubulaire externe (22) entourant l'organe interne, déplaçable par rapport à lui et élastiquement déformable entre:
   . un état ouvert pour lequel sa partie distale (34), de diamètre interne réduit, appuie sur la partie distale (28) de l'organe interne et qu'il garde tant que ce dernier est à l'état ouvert, et
   . un état fermé pour lequel sa partie distale (34) n'agit plus sur l'organe interne et qu'il prend lorsque ce dernier se met à l'état fermé.

## Description

La présente invention concerne un dispositif de sécurité pour la pointe d'une aiguille médicale qui vient d'être utilisée, soit seule soit en association avec une canule.

Il est maintenant connu, pour éviter toute contamination du personnel soignant ou d'entretien par une blessure occasionnée par une aiguille usagée, d'emprisonner sa pointe, dès l'instant où elle est sortie du corps du patient, dans une sorte de capuchon de protection.

Un dispositif de ce type est décrit, notamment, dans le document WO 99/10033, au nom du demandeur.

La présente invention a pour but de fournir un dispositif de protection amélioré qui répond, mieux que les dispositifs proposés jusqu'ici, aux exigences de simplicité, fiabilité, faible coût, faibles dimensions et légèreté.

De façon plus précise, l'invention concerne un dispositif de sécurité pour une aiguille médicale dotée d'une gorge ménagée à proximité de sa pointe, caractérisé en ce qu'il comporte:
- un organe tubulaire interne qui est à la fois déplaçable le long de l'aiguille jusqu'à sa pointe de manière à l'emprisonner et élastiquement déformable entre:
   . un état ouvert qu'il garde tant qu'il n'est pas au niveau de la pointe et pour lequel sa partie proximale, de diamètre interne réduit, enserre l'aiguille, et
   . un état fermé qu'il prend lorsqu'il arrive au niveau de la pointe et pour lequel sa partie proximale se trouve dans ladite gorge, et
- un organe tubulaire externe entourant l'organe interne, déplaçable sur lui et élastiquement déformable entre:
   . un état ouvert pour lequel sa partie distale, de diamètre interne réduit, appuie sur la partie distale de l'organe interne et qu'il garde tant que ce dernier est à l'état ouvert, et
   . un état fermé pour lequel sa partie distale n'agit plus sur l'organe interne et qu'il prend lorsque ce dernier se met à l'état fermé.

De préférence, l'organe tubulaire interne est rendu élastiquement déformable au moyen d'encoches pratiquées dans sa paroi à partir de son extrémité proximale sur une partie de sa longueur. De plus, il comporte une partie médiane dont le diamètre interne est légèrement plus grand que celui de l'aiguille, de manière à lui servir de guide pour qu'elle reste sensiblement coaxiale audit organe.

De préférence aussi, l'organe tubulaire externe est rendu élastiquement déformable au moyen d'encoches pratiquées dans sa paroi à partir de son extrémité distale sur une partie de sa longueur. De plus, il comporte une partie proximale dont le diamètre interne est adapté au diamètre externe de l'organe tubulaire interne.

L'invention concerne également un dispositif de sécurité pour une aiguille médicale destinée à être utilisée avec une canule, caractérisé en ce qu'il comporte:
- solidaire de l'extrémité de l'aiguille opposée à sa pointe, un organe tubulaire interne doté d'une gorge ménagée à sa partie distale et dont la partie proximale est de diamètre réduit, et
- solidaire de l'extrémité de la canule opposée à sa pointe, un organe tubulaire externe, déplaçable sur ce dernier et élastiquement déformable entre:
   . une position pour laquelle sa partie proximale, de diamètre réduit, est au niveau de la partie proximale de l'organe interne, la pointe étant alors à l'extérieur de la canule, et
   . une position pour laquelle sa partie proximale se trouve dans ladite gorge, la pointe étant alors emprisonnée dans l'extrémité de la canule,
   ledit organe externe étant élastiquement déformable entre un état fermé qu'il occupe lorsque sa partie proximale est dans ladite gorge ou au niveau de la partie proximale de l'organe interne et un état ouvert qu'il occupe entre ces deux positions.

Dans ce mode de réalisation, l'organe tubulaire externe est avantageusement rendu élastiquement déformable au moyen d'encoches pratiquées dans sa paroi à partir de son extrémité proximale sur une partie de sa longueur.

L'invention sera mieux comprise à la lecture de la description qui va suivre, faite en regard des dessins annexés, sur lesquels:
- les figures 1 et 2 représentent une aiguille médicale munie de son dispositif de sécurité dans la position qu'il occupe respectivement pendant et après l'utilisation de l'aiguille;
- les figures 3 et 4 représentent respectivement l'organe tubulaire interne et l'organe tubulaire externe du dispositif des figures 1 et 2;
- les figures 5 et 6 représentent une aiguille médicale et sa canule munies d'un dispositif de sécurité dans la position qu'il occupe respectivement pendant et après leur utilisation; et
- la figure 7 représente l'organe tubulaire externe du dispositif des figures 5 et 6.

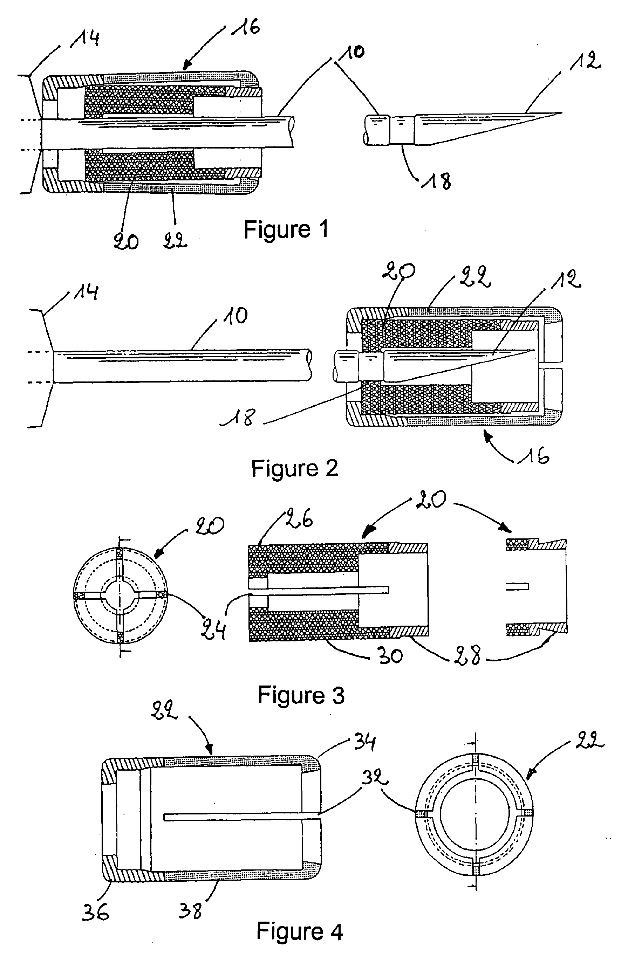

Les figures 1, 2, 3 et 4 montrent une aiguille médicale 10 dont une extrémité comporte une pointe 12 destinée à être introduite dans le corps d'un patient et dont l'autre extrémité est solidaire d'une garde 14 prévue pour être enfilée sur l'embout d'une seringue (non représentée).

Avant et pendant l'utilisation de l'aiguille 10, un dispositif de sécurité 16 est placé à proximité de la garde 14 (figure 1). Lors du retrait de l'aiguille, le dispositif 16 glisse jusqu'à sa pointe 12 pour l'emprisonner complètement et la rendre ainsi inoffensive (figure 2).

On notera, tout d'abord, que l'aiguille 10 comporte, à proximité de sa pointe 12, une gorge périphérique 18 ayant, typiquement, une profondeur d'environ 5/100 mm et dont l'utilité apparaîtra plus loin.

Le dispositif de sécurité 16 est constitué de deux tubes cylindriques concentriques en plastique 20 et 22, l'un interne et au contact de l'aiguille, l'autre externe et entourant le tube interne. Ces deux tubes, coaxiaux à l'aiguille 10, sont élastiquement déformables et font office de pinces, le tube externe 22 agissant sur le tube interne 20 qui agit lui-même sur l'aiguille.

Le tube interne 20 est déplaçable le long de l'aiguille depuis sa partie proximale adjacente à la garde 14 (figure 1) jusqu'à sa pointe 12 (figure 2). Comme le montre mieux la figure 3, ce tube est rendu élastiquement déformable entre un état ouvert (figure 1) et un état fermé (figure 2) au moyen de quatre encoches 24 pratiquées dans sa paroi, à 90° les unes des autres, à partir de son extrémité proximale sur une partie de sa longueur. Bien entendu, selon les dimensions du tube et le matériau dont il est constitué, le nombre d'encoches 24 peut être plus faible ou plus élevé que dans l'exemple donné ci-dessus.

Par ailleurs, le tube interne 20 comporte une partie proximale 26 à diamètre intérieur réduit qui, dans l'état ouvert, enserre l'aiguille tant qu'il n'est pas au niveau de sa pointe et, dans l'état fermé, pénètre dans la gorge 18 au moment où il arrive au niveau de la pointe.

Le tube interne 20 comporte, en outre, une partie distale 28 à grand diamètre intérieur et une partie médiane 30 dont le diamètre intérieur est légèrement plus grand que celui de l'aiguille 10 de manière à lui servir de guide pour qu'elle reste sensiblement coaxiale au tube. Enfin, l'extrémité de la partie distale 28 présente un diamètre externe légèrement réduit.

Le tube externe 22 entoure le tube interne 20 et peut se déplacer par rapport à lui entre une première position (figure 1) pour laquelle le tube interne, à l'état ouvert, n'est pas au niveau de la pointe 12 et une deuxième position (figure 2) pour laquelle le tube interne, à l'état fermé, se trouve au niveau de la pointe. Comme le montre mieux la figure 4, ce tube est rendu élastiquement déformable entre un état ouvert (figure 1) et un état fermé (figure 2) au moyen de quatre encoches 32 pratiquées dans sa paroi, à 90° les unes des autres, à partir de son extrémité distale sur une partie de sa longueur. Comme pour le tube interne 20, selon les dimensions du tube externe 22 et le matériau dont il est constitué, le nombre d'encoches 32 peut être plus faible ou plus élevé que dans l'exemple donné ci-dessus.

Par ailleurs, le tube externe comporte une partie distale 34 à diamètre intérieur réduit qui, lorsqu'il est à l'état ouvert et occupe sa première position (figure 1), appuie sur la partie distale 28 du tube interne 20.

Le tube externe 22 comporte, en outre, une partie proximale 36 dont le diamètre interne réduit est adapté au diamètre externe du tube interne 20, ainsi qu'une partie médiane 38 à grand diamètre.

En variante, comme le montre la partie droite de la figure 3, la partie distale 28 du tube interne 20 peut présenter un gorge inclinée évitant que dans sa première position, la partie distale 34 du tube externe 22 appuie sur le tube interne.

Le fonctionnement du dispositif 16 décrit en regard des figures 1 à 4 est le suivant.

Avant et pendant l'utilisation de l'aiguille 10, le dispositif de sécurité se trouve proche de sa garde 14 (figure 1). Le tube externe 22 est alors, à l'état ouvert, dans sa première position pour laquelle il appuie, par sa partie distale 34, sur la partie distale 28 du tube interne 20 qui est aussi à l'état ouvert et dont l'autre extrémité est bloquée à l'entrée de la partie proximale 36 du tube externe 22. La partie proximale 26 du tube interne 20 est alors suffisamment pressée contre l'aiguille pour éviter tout déplacement intempestif du dispositif 16.

Lorsque l'aiguille 10 est retirée du patient, le dispositif 16 est maintenu passivement ou activement (selon qu'il est monté sur un cathéter ou une aiguille) de manière à ce qu'il glisse jusqu'à ce que la partie proximale 26 du tube interne 20 tombe dans la gorge 18 (figure 2). Le tube interne se trouvant ainsi à l'état fermé, il permet au tube externe 22 de passer, lui aussi, à l'état fermé, dans sa deuxième position pour laquelle les parties proximales 26 et 36 sont en contact l'une avec l'autre, tandis que les partie distales 28 et 34 ne le sont plus. La pointe 12 est alors emprisonnée dans le dispositif qui se trouve arrimé sur elle du fait que, d'une part, la partie proximale 26 du tube interne 20 est maintenue dans la gorge 18 par la partie proximale 36 du tube externe et que, d'autre part, la partie distale 34 du tube externe 22 interdit le mouvement relatif des deux tubes.

Lorsque le dispositif 16 qui vient d'être décrit est utilisé sur une aiguille hypodermique (et non un cathéter), il peut être avantageux de l'entourer d'un manchon métallique rigide (non représenté) qui est chassé sur l'extrémité proximale du tube externe 22 et sert à protéger son fonctionnement de pressions asymétriques externes exercés par l'opérateur au moment du retrait de l'aiguille.

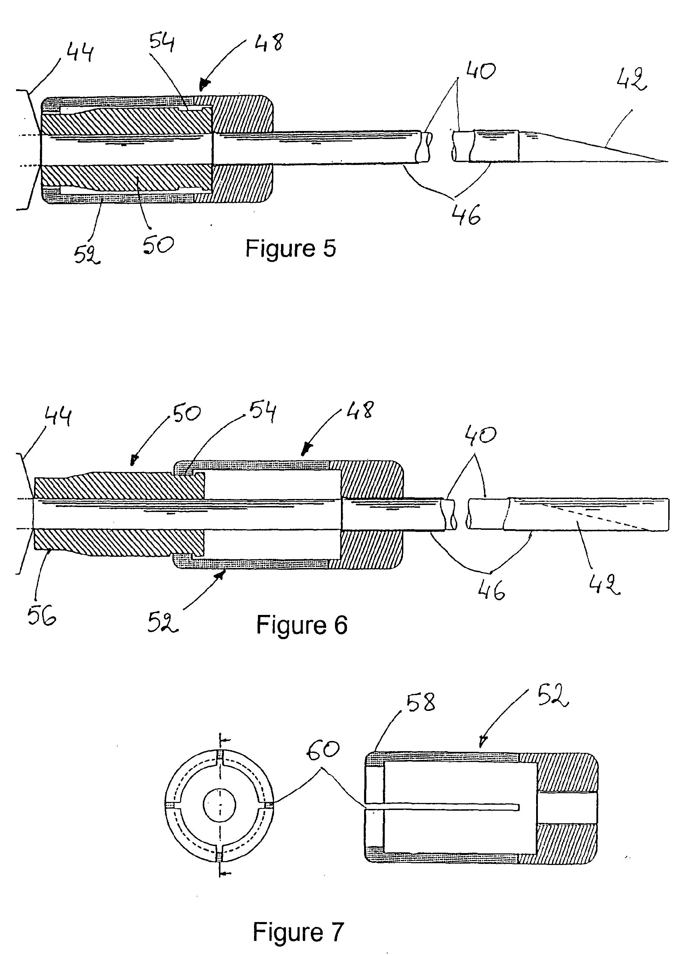

On se référera maintenant aux figures 5, 6 et 7 qui montrent une aiguille médicale 40 dont une extrémité comporte une pointe 42 et dont l'autre extrémité est solidaire d'une garde 44 prévue pour être enfilée sur l'embout d'une seringue (non représentée). A la différence du mode de réalisation précédemment décrit, l'aiguille 40 est disposée à l'intérieur d'une canule métallique 46.

Un dispositif de protection 48 est constitué de deux tubes cylindriques concentriques 50 et 52, l'un interne et solidaire de l'extrémité de d'aiguille 40 opposée à sa pointe, l'autre externe et solidaire de l'extrémité de la canule 46 opposée à sa pointe.

Afin de réduire le nombre de pièces, le tube interne 50 peut être moulé d'une seule pièce avec la garde 44.

Avant et pendant l'utilisation de l'aiguille 40, le tube interne 50 est complètement à l'intérieur du tube externe 52 (figure 5). Lors du retrait de l'aiguille, la canule 46 restant, par adhérence ou succion, dans le corps du patient, le tube interne 50 est extrait quasi complètement du tube externe 52 de manière à ce que la pointe 42 se trouve emprisonnée dans la canule 46 et rendue ainsi inoffensive avant même la sortie de l'aiguille (figure 6).

Le tube interne 50, qui reçoit l'aiguille sur toute sa longueur, est muni, à sa partie distale, d'une gorge 54 ayant, typiquement, une profondeur d'environ 2/10 mm. Par ailleurs, sa partie proximale 56 est de diamètre réduit.

Le tube externe 52, solidaire de la canule 46 seulement à sa partie distale, est déplaçable avec une faible friction par rapport au tube interne 50 entre une position (figure 5) pour laquelle sa partie proximale 58, de diamètre réduit, est au niveau de la partie proximale 56 du tube interne 50, la pointe 42 étant alors à l'extérieur de la canule 46, et une position (figure 6) pour laquelle sa partie proximale 58 se trouve dans la gorge 54, la pointe 42 étant alors emprisonnée dans l'extrémité de la canule 46.

Le tube externe 52 est élastiquement déformable entre un état fermé qu'il occupe lorsque sa partie proximale 58 est au niveau de la partie proximale 56 ou dans la gorge 54 du tube interne 50 et un état ouvert qu'il occupe entre ces deux positions.

Comme le montre mieux la figure 7, le tube externe 52 est rendu élastiquement déformable entre son état ouvert et son état fermé au moyen d'encoches 60 pratiquées, à partir de son extrémité proximale 58, sur une partie de sa longueur.

Le fonctionnement du dispositif 48 décrit en regard des figures 5 à 7 est le suivant.

Avant et pendant l'utilisation de l'aiguille 40 (figure 5), le tube externe 52 est, à l'état fermé, dans sa position pour laquelle il appuie, par sa partie proximale 58, sur l'extrémité proximale 56 du tube interne 50 et se trouve ainsi arrimé sur elle, solidairement de la canule 46, de manière à éviter tout déplacement intempestif.

A l'instant du retrait de l'aiguille 40, la canule 46 restant en place (figure 6), le tube interne 50 sort du tube externe 52 qui passe à l'état ouvert. Le tube interne glisse alors jusqu'à ce que la partie proximale 58 du tube externe tombe dans la gorge 54. La pointe 42 se trouve emprisonnée dans la canule 46 et le tube externe 52, revenu à l'état fermé dans la gorge 54, est alors suffisamment arrimé au tube interne 50 pour empêcher un déplacement de l'un par rapport à l'autre, qui pourrait faire ressortir la pointe de la canule.

## Revendications

1. Dispositif de sécurité (16) pour une aiguille médicale (10) dotée d'une gorge (18) ménagée à proximité de sa pointe (12), **caractérisé en ce qu'**il comporte:
- un organe tubulaire interne (20) déplaçable le long de l'aiguille jusqu'à sa pointe de manière à l'emprisonner et élastiquement déformable entre:
. un état ouvert qu'il garde tant qu'il n'est pas au niveau de la pointe et pour lequel sa partie proximale (26), de diamètre interne réduit, enserre l'aiguille, et
. un état fermé qu'il prend lorsqu'il arrive au niveau de la pointe et pour lequel sa partie proximale (26) se trouve dans ladite gorge (18), et
- un organe tubulaire externe (22) entourant l'organe interne, déplaçable par rapport à lui et élastiquement déformable entre:
. un état ouvert pour lequel sa partie distale (34), de diamètre interne réduit, appuie sur la partie distale (28) de l'organe interne et qu'il garde tant que ce dernier est à l'état ouvert, et
. un état fermé pour lequel sa partie distale (34) n'agit plus sur l'organe interne et qu'il prend lorsque ce dernier se met à l'état fermé.

2. Dispositif de sécurité selon la revendication 1, **caractérisé en ce que** l'organe tubulaire interne (20) est rendu élastiquement déformable au moyen d'encoches (24) pratiquées dans sa paroi à partir de son extrémité proximale sur une partie de sa longueur.

3. Dispositif de sécurité selon l'une des revendications 1 et 2, **caractérisé en ce que** l'organe tubulaire interne (20) comporte une partie médiane (30) dont le diamètre interne est légèrement plus grand que celui de l'aiguille, de manière à lui servir de guide pour qu'elle reste sensiblement coaxiale audit organe.

4. Dispositif de sécurité selon la revendication 1, **caractérisé en ce que** l'organe tubulaire externe (22) est rendu élastiquement déformable au moyen d'encoches (32) pratiquées dans sa paroi à partir de son extrémité distale sur une partie de sa longueur.

5. Dispositif de sécurité selon l'une des revendications 1 et 4, **caractérisé en ce que** l'organe tubulaire externe (22) comporte une partie proximale (36) dont le diamètre interne est adapté au diamètre externe de l'organe tubulaire interne (20), afin de lui interdire de sortir de la gorge (18).

6. Dispositif de sécurité (48) pour une aiguille médicale (40) destinée à être utilisée avec une canule (46), **caractérisé en ce qu'**il comporte:
- solidaire de l'extrémité de l'aiguille opposée à sa pointe (42), un organe tubulaire interne (50) doté d'une gorge (54) ménagée à sa partie distale et dont la partie proximale (56) est de diamètre réduit, et
- solidaire de l'extrémité proximale de la canule, un organe tubulaire externe (52) déplaçable par rapport à l'organe interne (50) entre:
. une position pour laquelle sa partie proximale (58), de diamètre réduit, est au niveau de la partie proximale (56) de l'organe interne, la pointe (42) étant alors à l'extérieur de la canule (46), et
. une position pour laquelle sa partie proximale (58) se trouve dans ladite gorge (54), la pointe (42) étant alors emprisonnée dans l'extrémité de la canule (46),
ledit organe externe (52) étant élastiquement déformable entre un état fermé qu'il occupe lorsque sa partie proximale (58) est dans ladite gorge (54) ou au niveau de la partie proximale (56) de l'organe interne et un état ouvert qu'il occupe entre ces deux positions.

7. Dispositif de protection selon la revendication 6, **caractérisé en ce que** l'organe tubulaire externe (52) est rendu élastiquement déformable au moyen d'encoches (60) pratiquées dans sa paroi à partir de son extrémité proximale sur une partie de sa longueur.
